# EUROPEAN PATENT SPECIFICATION

(11) **EP 3 752 827 B1**
(45) Date of publication and mention of the grant of the patent: **24.08.2022**
(21) Application number: 19704460.5
(22) Date of filing: 16.01.2019
(51) Int. Cl.: A61B 90/00, G02B 21/36

(54) **APPARATUS FOR THE EX-VIVO INTRAOPERATIVE ANALYSIS OF BIOLOGICAL TISSUE SAMPLES**
VORRICHTUNG ZUR INTRAOPERATIVEN EX-VIVO-ANALYSE VON BIOLOGISCHEN GEWEBEPROBEN
APPAREIL POUR L'ANALYSE PEROPÉRATOIRE EX-VIVO D'ÉCHANTILLONS DE TISSU BIOLOGIQUE

(30) Priority: 16.01.2018 IT 201800001108
(43) Date of publication of application: 23.12.2020
(73) Proprietor: Scuola Superiore Sant'Anna, 56127 Pisa (IT); Universita' Degli Studi di Firenze, 50134 Firenze (IT)
(72) Inventor: CAPINERI, Lorenzo, 50139 Firenze (IT); FROSINI, Francesco, 50139 Firenze (IT); MAZZONI, Marina, 50139 Firenze (IT); BULLETTI, Andrea, 50139 Firenze (IT); MENCIASSI, Arianna, 56127 Pisa (IT); ODDO, Calogero Maria, 56127 Pisa (IT); MASSARI, Luca, 56127 Pisa (IT); CIUTI, Gastone, 56127 Pisa (IT)
(74) Representative: Leotta, Antonio
(86) International application number: PCT/IB2019/050335
(87) International publication number: WO 2019/142109

(56) References cited:
- WO-A1-2010/129773
- WO-A2-2009/039428
- NAIDU ANISH S ET AL: "A Breakthrough in Tumor Localization: Combining Tactile Sensing and Ultrasound to Improve Tumor Localization in Robotics-Assisted Minimally Invasive Surgery", IEEE ROBOTICS & AUTOMATION MAGAZINE, IEEE SERVICE CENTER, PISCATAWAY, NJ, US, vol. 24, no. 2, 9 May 2017 (2017-05-09), pages 54-62, XP011652683, ISSN: 1070-9932, DOI: 10.1109/MRA.2017.2680544 [retrieved on 2017-06-13] cited in the application
- RIBBACK SILVIA ET AL: "Virtual slide telepathology with scanner systems for intraoperative frozen-section consultation", PATHOLOGY - RESEARCH AND PRACTICE, vol. 210, no. 6, 4 March 2014 (2014-03-04) , pages 377-382, XP028649103, ISSN: 0344-0338, DOI: 10.1016/J.PRP.2014.02.007 cited in the application

## Description

### TECHNICAL FIELD

The present invention relates to the field of technological devices in the medical environment, referring in particular to integrated systems for the analysis of tissue samples for bioptic investigations.

### STATE OF THE ART

Intraoperative analysis of biological tissue samples takes place during the course of a surgery. Usually when we talk about intraoperative histological examination we mean the one performed on a patient during surgery and aimed at identifying tumor lesions in order to allow the immediate removal. With the known techniques and apparatus it is possible to proceed only to the identification of tumor inclusions but not to their precise characterization.

The intraoperative histological examination is the technique that allows to perform a histological study on a biopsy sample taken during a surgery. Through this examination it is possible to provide immediate information to the surgical team, which can thus better define the intervention and the surgery procedure itself. For example, this information could be fundamental to define the margins of a resection, to define the type of a lesion (benign vs. malignant, infiltrated vs. non-infiltrated), to define the invasiveness of the intervention as a whole.

This techniques provides that during the surgical procedure it is resected a portion of biological material, which is sent to the pathological anatomy laboratory, which performs the analysis and sends the result to the surgical team. Everything is done in a very short time (20-30 min.). This approach provides that in the pathological anatomy laboratory there are specialized doctors and health technicians: the first perform the selection of the useful parts of the anatomical piece (sampling) and the reading/refertation of the slide; the second take care of preparing the sample for reading (cutting, coloring and preparation of the slide).

The technological evolution of the such methodology has seen the introduction of Information & Communication Technology with the aim of reducing information transit times. In particular, the remotization of the response allowed to speed up the time needed to provide the information to the surgical team, once the pathological analysis has been completed.

The literature highlights that telepathology and remotization of histological examination have reached a good maturity level. In the work of Ribback S. et al (2014) "Virtual slide telepathology with scanner systems for intraoperative frozen-section consultation, Pathology Research and Practice" they talk about telepathology systems highlighting the complications related to the transport of samples from the operating room to the laboratory. In some cases, the two places may be too far from each other making intraoperative analysis practically impossible. The author illustrates a system that can virtualize the biopsy using a scanner, but making necessary a laboratory of pathological anatomy near the operative compartment. The remotization of information is linked only to the reading phase of the result image and to the return of the response to the operating room.

With regard to the in-vivo techniques, the method traditionally used for the identification of tumors under the surface is that of the direct palpation of the organ made by the surgeon who uses his sense of touch. However, this technique can only be used in the case of open surgery, to which risks with infections and morbidity are associated. For this reason, Robotics-Assisted Minimally Invasive Surgery (RAMIS) is increasingly used, which, however, prevents a direct palpation by the surgeon. It has been developed an intraoperative use, with RAMIS techniques, of an ultrasound probe to locate a subsurface tumor, but the images obtained are of poor quality and prevent a reliable diagnosis. The use of tactile sensor array has also been explored to allow a virtual tactile feedback in minimally invasive surgery.

The system indicated in the article "A Breakthrough in Tumor Localization: Combining Tactile Sensing and Ultrasound to Improve Tumor Localization in Robotics-Assisted Minimally Invasive Surgery" by Anish S. Naidu et al. (IEEE Robotics & Automation Magazine, 2017) anticipates the use of two types of sensors, tactile and ultrasound, mounted on a da Vinci type surgery robot to perform an in-vivo intraoperative histological examination. The system provides both a tactile sensor and an ultrasound sensor arranged opposite to each other on its end effector mounted on an articulated wrist that allows the palpation of the tissue with wide adjustability. The tactile sensor is an array of piezoresistive or capacitive sensors and the ultrasound sensor is an array of linear transducers. The sensors are associated with the same end effector, but are not integrated with each other, to be used alternatively during the resection intervention according to the surgeon's will. This system is dedicated and designed for in vivo intraoperative use and therefore does not use any remotization. Furthermore, the purpose of the system is that of identification and it is not highlighted the presence of histological analysis. Besides, the use of linear sensor arrays implies a large contact surface on the organic tissue that can induce errors due to the imperfect perpendicularity of all the single sensors with respect to the contact surface and small differences in distance between the sensors and the contact surface itself. Finally, systems that have both tactile sensors and ultrasound sensors have never been used for the ex-vivo intraoperative histological analysis.

Another example of apparatus for performing in-vivo procedures is the one described in WO 2009/039428 which relates to an apparatus for the administration of anti-tumor radiotherapy by means of a robotic arm. The document also mentions the possibility of combining in the device of the invention PET/CT/MR technologies and direct imaging such as imaging with cameras, intraoperative imaging using ultrasound and tactile sensors to accurately identify the area to be treated, the treatment depth and the correct radiation dose. However, even this device, apart from concerning in-vivo and not ex-vivo procedures, only mentions the possibility of using in the same object multiple detection technologies such as imaging, ultrasound and tactile, but does not describe in any way the implementation methods, nor even the interaction modalities to obtain synergistic effects.

As regards instead the ex-vivo intraoperative analysis systems, which is the object of the present invention, US2016313252 describes a system that allows to perform a histological analysis by means of optical scanning of a sample whose results are sent remotely to the pathologist. This system allows to remote the analysis in a non-destructive way for the sample but is not able to detect the presence or characterize any inclusions.

A device not designed for the intraoperative analysis, instead, is the one described in CN105352931, which provides for the detection of tumor cells or other pathological cells through the use of a laser light emission system with fluorescence detection which also includes an ultrasound probe connected to a data processing system. As already highlighted, this system is not suitable for an intraoperative use.

### SUMMARY OF THE INVENTION

The object of the present invention is therefore to improve the telepathology systems and to improve the ex-vivo intraoperative analysis process by providing an integrated system that allows not to physically send the material to the pathological anatomy laboratory but instead uses a suitable room, located within the operating area or near it.

Another object of the present invention is to propose a system that ensures an objective and reproducible ex-vivo intraoperative analysis, by means of a complete scan of the resected samples, carried out in a controlled environment.

Another object of the present invention is to propose an apparatus for the ex-vivo intraoperative analysis of biological tissue samples that is able to operate the analysis of a sample in a fully automatic mode allowing access to the results in remote mode, in particular to a pathologist.

Another object of the present invention is to propose an apparatus for the ex-vivo intraoperative analysis of biological tissue samples which gives very reliable results also thanks to the simultaneous integration of several analysis methods.

Another object of the present invention is to propose an apparatus for ex-vivo intraoperative histological analysis that integrates tactile sensors, imaging and ultrasound detection techniques that work synergistically to automate the analysis process and provide more reliable results.

Another object of the present invention is to propose an apparatus for the ex-vivo intraoperative histological analysis which integrates tactile sensors and ultrasound detection techniques integrated in an optimized way with high precision and reliable technologies but at low production and operational cost.

These and other objects of the present invention are achieved by an apparatus for the ex-vivo intraoperative analysis of a biological tissue sample resected from a patient during surgery, said apparatus comprising:
- supporting means, to which said sample can be stably associated, comprising a platform provided with a flat surface adapted to receive the sample to be analysed,
- data acquisition bodies comprising at least a sensor element,
- moving members of the supporting means with respect to the data acquisition bodies,
- a control unit for receiving data from the acquisition bodies, for processing them and for controlling the acquisition bodies themselves and the moving members, in which the acquisition bodies comprise:
- ultrasound sensors,
- tactile sensors,
- vision sensors,
wherein the sensor element provides a free end forming a contact surface, facing the flat surface of the supporting means, suitable for abutting said biological tissue sample, said contact surface being a detection surface of the tactile sensors and the same emission and reception surface of said ultrasound sensors, and wherein the control unit is configured to automatically control the moving members and the ultrasound sensor as a function of data received from the vision sensors and/or from the tactile sensors in such a way to control the acquisition of data by the ultrasound sensors and tactile sensors and to elaborate the acquired data for a characterization of the sample itself.

The systemic integration between vision sensors, tactile sensors and ultrasound sensors allows to automate the biological tissue sample analysis process since the same vision sensors and tactile sensors are used to characterize the sample as well as to synchronize and adjust the movement of the moving members that allow the sample to be scanned.

In addition, the simultaneous use of various types of sensor technologies makes it possible to verify the correlation between the results and therefore to improve the reliability of the same.

The fact that the tactile sensor and the ultrasound sensor are integrated into a same sensor element makes the structure simpler and therefore the costs of the apparatus is reduced and it allows to obtain in the same identical point and simultaneously both a tactile and ultrasound analysis of the sample.

Advantageously, the ultrasound sensors are constituted by a single ultrasound sensor and the tactile sensors consist of a single tactile sensor, both associated to the same sensor element which is a rod-shaped element with a needle-like free end so that the contact surface is small in size with respect to the biological tissue sample size. Furthermore, the control unit is configured to: automatically control the moving members, the ultrasound sensor and the tactile sensor in such a way that the aforesaid contact surface indents the biological tissue sample in correspondence of a plurality of points to be analysed in a sequence; and to command the acquisition of data by the ultrasound sensor and the tactile sensor at each point of the plurality of points to be analysed.

The use of a single ultrasound sensor and a single tactile sensor in a needle sensor element allows to perform a measurement that is not affected by misalignment errors between sensors and variations in the distance from the sample. In addition, the ultrasound analysis is carried out following an indentation at a controlled force thus allowing the same indentation force to be applied to the whole sample and consequently improving the possibility of comparison between the results.

Alternatively, the ultrasound sensors comprise an array of ultrasound transducers and the tactile sensors comprise an array of tactile sensors, both associated with the sensor element. This embodiment involves a contact surface of the sensor element of greater dimensions, for example with a surface extension comparable to that of the sample to be analysed. In this case the above-mentioned advantages relating to the presence of a needle-like contact element are lost and however may be preferable in some implementations since the scanning of the entire sample can take place with a single contact movement between the sensor element and the sample, or with a limited number of contact movements, thus significantly reducing the scanning time.

Advantageously, said vision sensors comprise at least one camera, said control unit being configured to automatically process images detected by said vision sensors in order to identify a border of the sample to be analysed and the plurality of points to be analysed within an area inside said border. Preferably, the vision sensors comprise a plurality of high-definition cameras, the control unit being configured to process the images detected by the cameras to identify the three-dimensional border of the sample.

The same vision sensors are used both to automate the analysis process by recognizing the sample shape and position and to obtain a sample characterization based on imaging techniques.

Advantageously, the control unit is configured to, following the definition of said plurality of points to be analysed:
- command the automatic indentation in sequence, by said sensor element, of each of said points to be analysed by actuation of said moving members,
- at each of the point to be analysed, acquire data by the tactile sensor and the ultrasound sensor,
- process the data acquired by the tactile sensor and the ultrasound sensor independently from each other to determine parameters of physical characterization of said sample.

The control unit, after having determined a mesh of points in correspondence of which tactile and ultrasound measurements are to be carried out, is programmed to command the moving members and the activation of the tactile and ultrasound sensors so that the whole scanning process of the sample takes place automatically so that the analysis process is extremely repeatable and objective.

Still advantageously, the control unit is configured to process the data acquired by the vision sensors independently from the data acquired by the tactile sensor and the ultrasound sensor to determine the same physical characterization parameters of the sample.

Advantageously, the apparatus comprises a portal-like bearing structure that defines therewithin a volume of analysis, said vision sensors being firmly associated with the portal-like bearing structure to shoot the volume of analysis, said sensor element being firmly associated with the upper cross-members of the portal-like bearing structure to protrude downwards into the volume of analysis, the supporting means comprising a platform provided with a flat upper surface adapted to receive the sample on it within the volume of analysis, with the moving members supporting the platform consisting of a motorized group with four controlled axes, three of which are of translation and one of rotation. Preferably, the portal-like bearing structure comprises upper cross-members, lower cross-members and uprights, with the moving members mounted to the lower cross-members. Moreover, the moving members comprise longitudinal horizontal slide guides, cross horizontal slide guides, a lifting mechanism, and a rotary cylinder with vertical axis, so that the moving members allow the sample to be moved and oriented to any position within the volume of analysis with the planar surface of the platform kept in horizontal planar configuration.

Thanks to the portal-like bearing structure and the configuration of the moving members, there is a high precision of relative positioning between the sample and the sensor system and it is possible to perform the scanning process at high speed.

Advantageously, the ultrasound sensors and the tactile sensors are included in a same structure fixed below the upper cross-members and comprising a load cell to which it is rigidly fixed to protrude downwards the sensor element the free end of which constitutes the contact surface facing the upper planar surface of the supporting means.

Thanks to the fact that the ultrasound probe is rigidly connected to a load cell, the sensing surface of the ultrasound sensor also becomes the sensing surface of the tactile sensor and therefore there is a perfect integration between the two sensors that can simultaneously detect data in the same point of the sample. Furthermore, the use of a load cell as the tactile sensor allows reduced operating costs since the load cell does not come into direct contact with the sample to be analyzed.

Advantageously, output means of the results of the analysis performed by the apparatus comprise a haptic interface actuated as a function of the information relating to the physical characterization parameters of the sample transmitted from the control unit.

### BRIEF DESCRIPTION OF THE DRAWINGS

These and other advantages and characteristics associated with the apparatus of the present invention, will become, however, more easily understood through the illustration of non-limiting exemplifying embodiments, as described below with the aid of the enclosed drawings, in which:
- Fig. 1 shows a schematic representation of the main components of the apparatus of the invention,
- Fig. 2 shows an axonometric view of an embodiment of the apparatus of the invention,
- Fig. 3 shows a flow chart illustrating the operation of the apparatus of the invention,
- Fig. 4 shows a detail of the embodiment of Fig. 2 during an analysis step,
- Fig. 5 shows a detail of the embodiment of Fig. 2 during a further analysis step,
- Fig. 6 shows a top view of a biological tissue sample analyzed in the apparatus of the invention.

### DESCRIPTION OF THE PREFERRED EMBODIMENTS

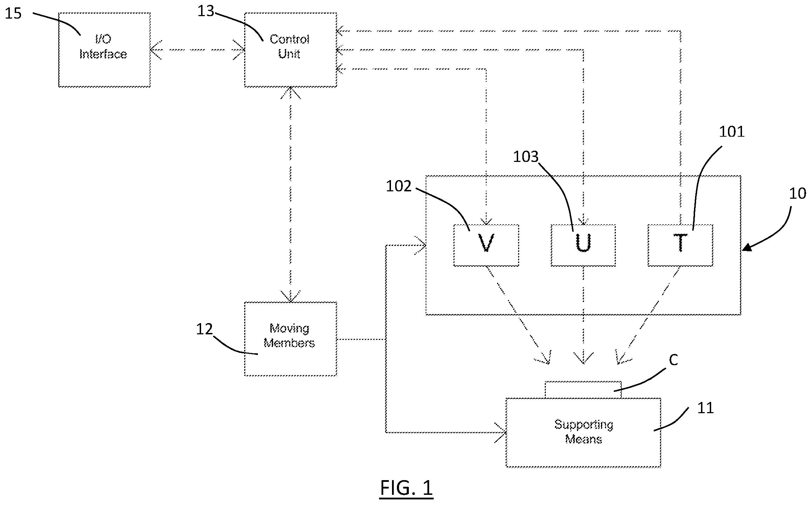

With reference to the schematic representation of Fig. 1, an apparatus 1 according to the present invention comprises data acquisition bodies, 10, supporting means, 11, to which a biological tissue sample, C, to be analysed can be firmly associated, moving members, 12, adapted to move the supporting means 11, and therefore the histological sample C, relative to the data acquisition bodies 10, a control unit, 13, and interface members, 15, to allow the input of working parameters of the apparatus and their visualization as well as of the results of the analysis performed. The data acquisition bodies 10 comprise tactile sensors, 101, vision sensors, 102, and ultrasound sensors, 103, each of which is directly connected to the control unit 13 to send and/or receive data about the operations they perform.

The control unit 13 is suitable for controlling the data acquisition bodies 10 so that after processing the data acquired by the latter it is able to control the moving members 12 in such a way as to be able to manage the relative position between the biological tissue sample C and the acquisition bodies 10. In particular, the control unit 13 is suitable for automatically controlling said moving members 12 and the ultrasound sensors 103 as a function of data received by the vision sensors 102 and by the tactile sensors 101 so as to control the acquisition of data by the ultrasound sensors 103 and tactile sensors 101 in correspondence with a plurality of points to be analysed of the biological tissue sample C and process the acquired data to determine a characterization of the sample C itself.

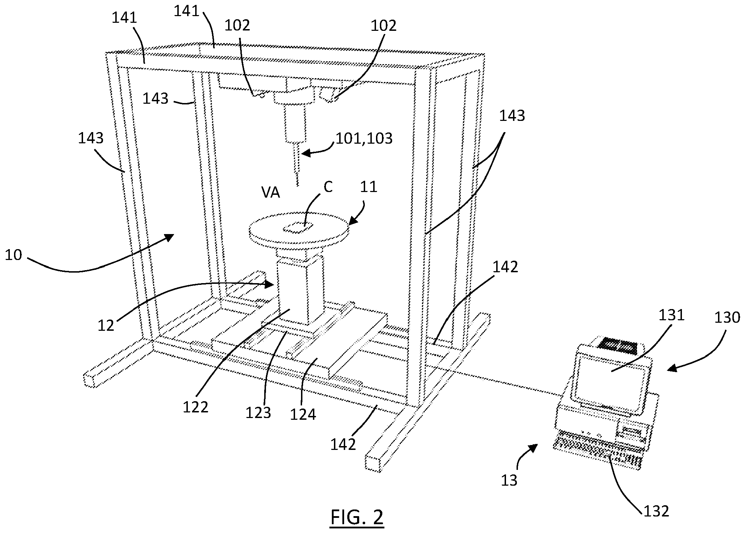

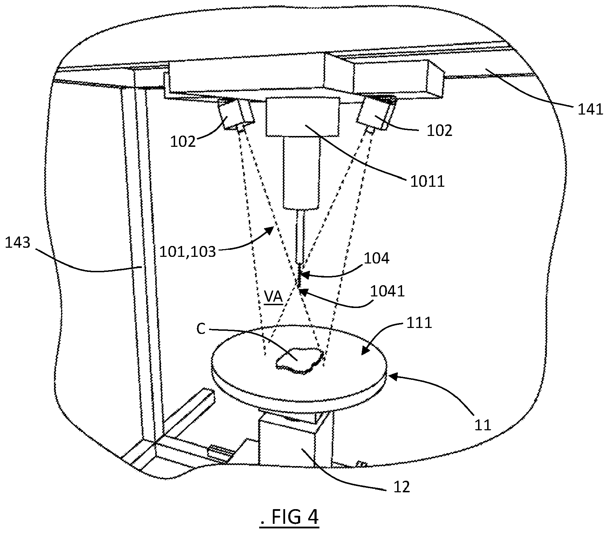

In Fig. 2 and in greater detail in Fig. 4, it is visible the apparatus 1 for the analysis of histological samples C according to the invention in an embodiment thereof as a whole comprising a self-supporting structure, 14, made of rigid upper cross members 141, lower cross members 142, and uprights 143, joined together to form a frame that identifies therewithin a volume of analysis, VA. At the lower cross members 142, moving members 12 are mounted which are motorized with four controlled axes above which are in turn fixed the supporting means 11 of the biological tissue sample C, constituted by a circular platform provided with an upper planar surface, 111, suitable for receiving the biological tissue sample C resting on it, so that the latter is supported substantially at the center of the volume of analysis VA. On the other hand, at the upper cross members 141, vision sensors 102, of the camera type, set to shoot the volume of analysis VA, ultrasound sensors 103 and tactile sensors 101 are arranged. The vision sensors 102 in the embodiment shown are made up of two high-resolution cameras arranged to shoot the volume of analysis VA from two different angles, thus making possible a three-dimensional detection of the volume of analysis. The ultrasound sensors 103 are constituted by a single ultrasound sensor and the tactile sensors 101 by a single tactile sensor which are included in a same structure fixed below the upper cross members 141 and comprising a load cell 1011, to which it is in turn rigidly fixed, for protruding downwards into the volume of analysis VA, a sensor element 104, in the shape of a rigid rod, a needle-like free end 1042 of which provides a contact surface, 1041, small in size with respect to the dimensions of the biological tissue sample C, which faces the upper planar surface 111 of the supporting means 11 and is therefore suitable for indenting the biological tissue sample C. Thanks to the rigid connection between the load cell 1011 and the sensor element 104, the force acting on the contact surface 1041 is detected by the load cell 1011 so that the contact surface 1041 constitutes a sensing surface of the tactile sensor 101. The sensor element 104 is constituted by the body of a rod-shaped ultrasound probe with a needle-like free end 1042, so that the contact surface 1041, very small as it has an area of a few mm², represents a detection surface of the tactile sensor 101 and at the same time a surface of emission and reception of ultrasound signals of the ultrasound sensor 103 which can be for example an ultrasound probe of the type comprising a single crystal or a plurality of piezoelectric crystal elements or other piezoelectric material, arranged in matrices, comb, cubic and/or multilayer. In practice, in the embodiment shown, thanks to the fact that a rod-shaped ultrasound probe with a needle-like free end is rigidly mounted on the load cell 1011, the working end of said ultrasound probe constitutes the contact surface 1041 which is therefore both an emission and reception surface of the ultrasound sensor 103 and a sensing surface of the tactile sensor 101, thereby guaranteeing to the tactile analysis a limit spatial resolution defined by the dimensions of this contact surface.

The moving members 12 are motorized with four controlled axes provided with longitudinal horizontal slide guides 124, cross horizontal slide guides, 123, a rotary cylinder with vertical axis 122 and a lifting device (not shown). The moving members 12 allow translation along three axes, two horizontal and one vertical, and the rotation around the vertical axis. In this way the sample C can be arranged and oriented in any configuration, included in the volume of analysis VA, in which the upper planar surface 111 of the supporting means 11 is kept in a planar configuration orthogonal to the vertical axial direction of extension of the rod-shaped sensor element 104, and facing the detection direction of the vision sensors 102.

The moving members 12 and the data acquisition bodies 10 are connected to the control unit 13 which is advantageously implemented in a computing appliance comprising hardware and software suitable for controlling the various acquisition bodies and the moving members, the sampling at high frequency, advantageously between 1 GHz and 2 GHz, and the computational and memorization capacities necessary to allow processing and storage of data in the working time of the apparatus 1. A PC, 130, or other computer means, is advantageously integrated with the apparatus and comprises input and output interfaces 15 such as a keyboard, 132, and a screen, 131, which allow the local interaction with the apparatus 1 in order to display the data acquired and processed, to set the working parameters and to display the results of the analysis.

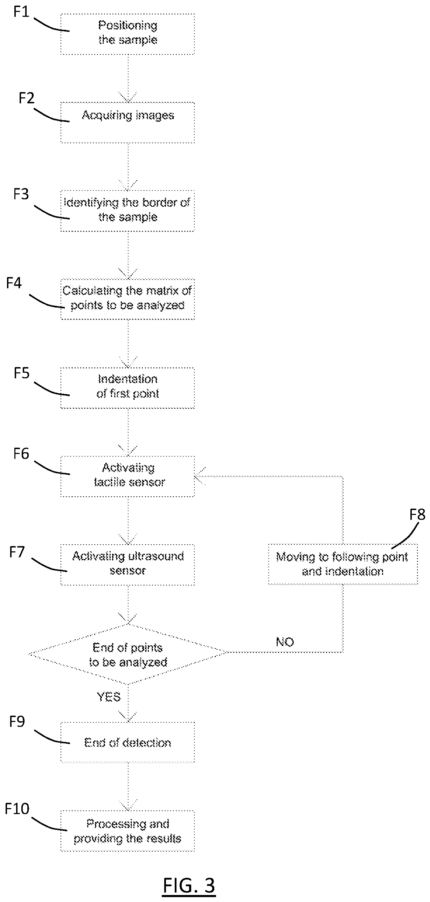

The operation of the above described apparatus 1 provides a sequence of steps schematically represented in the flow chart of Fig. 3 and included between the positioning, F1, of the biological tissue sample C on the supporting means 11 by the operator, and the provision of the results, F10, of the characterization of the biological tissue sample C itself. Each phase between F1 and F10 can take place automatically without the intervention of an operator and therefore quickly and without the need for a pathologist to be present in the operating room.

During a surgery on a patient, a biological tissue sample C is taken from the patient's body by resection, for example by radiofrequency or microwaves. As conventionally, the biological tissue sample C, before being placed on the supporting means 11, is subjected to a preparatory treatment comprising cutting, fixation and coloring.

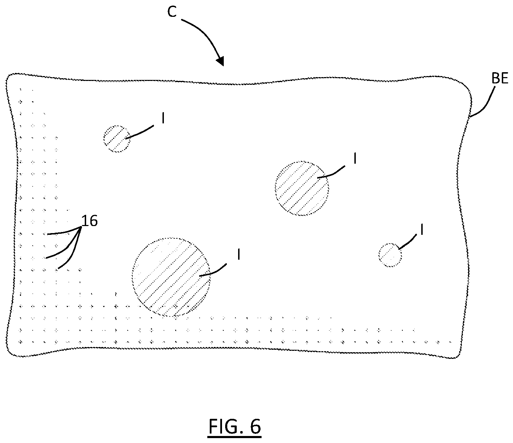

As shown in the detail view of Fig. 4 and in Fig. 6, once the sample C is positioned on the supporting means 11, the analysis procedure can be started, beginning with the acquisition of the images, F2, of the volume of analysis VA by the vision sensors 102, controlled by the control unit 13. In this phase of acquisition of the images F2, the vision sensors 102 perform an optical detection and the images are processed by the control unit 13 in order to identify the outer border, BE, of the biological tissue sample C, phase F3. Once the border BE of sample C has been identified, the control unit executes an algorithm which automatically determines, within the outer border BE, a plurality of points, 16, in correspondence of which the analysis is to be performed and are calculated relative indentation paths, step F4, that is to say the succession of relative movements between the sensor element 104 and the sample C which must be performed to bring the contact surface 1041 in correspondence of each point to be analysed 16. In this way it is made a mapping for significant points of the volume contained within the outer border BE and the data relating to this mapping are used by the control unit 13 to manage the actuation of the moving members 12.

The aforementioned steps for identifying F3 the border of the sample C and calculating F4 a matrix of points to be analysed can be performed completely automatically based on predefined operation parameters of the apparatus, or can be guided manually by a pathologist remotely connected to the device, which is able to remotely view the images acquired by the vision sensors 102 and to remotely set operating parameters of the apparatus such as manually defining the border BE of the sample C or the density of the matrix of points to be analysed 16.

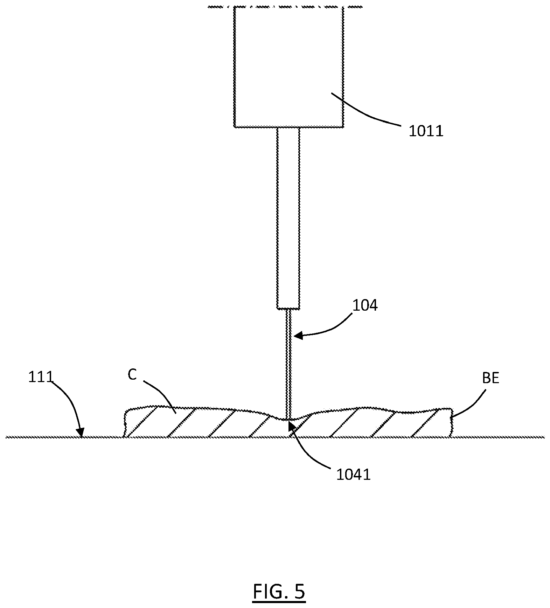

Once the matrix of points to be analysed 16 has been defined and the sequence of paths are calculated, it is started the indentation, F5, of the first point of the aforementioned matrix of points to be analysed by actuating the moving members 12 which approach the sample C in the direction of the axis of the tactile sensors 101 until a contact is reached between the sample C and the contact surface 1041. With reference to the detailed side view of Fig. 5, the indentation of the sample C by the sensor element 104 takes place until a limit force value is measured by the load cell 1011, step F6. This last limit force value, in fact, is set beforehand and when this value is reached, the control unit 13 commands a stop or an active control of the moving members 12 to adjust the contact force. In the position reached by the contact surface 1041 a first detection istance by the tactile sensor 101 and the ultrasound sensor 103 occurs. A tactile characterization of the point to be analysed 16 is obtained by processing the signal difference as a function of the entity of the advancement measured by the load cell 1011 between the start of the indentation and the stop when the limit force value is reached. Moreover, once the indentation movement is stopped, the control unit 13 controls the activation of the ultrasound sensor 103 so that from the contact surface 1041 occurs the emission, F7, of ultrasound signals and the consequent reception of the quota of the reflected signal which, processed by the control unit 13 according to suitable algorithms, allows a physical characterization of the sample C in that determined point to be analysed 16.

Once the data acquisition of both the tactile sensor 101 and the ultrasound sensor 103 in the first point has been performed, the control unit 13 controls the moving members 12 to exit from the first indentation point, move over the second point to be analyzed of the previously defined matrix of pointsx and start indenting, F8, the second point, and then repeating the F6 and F7 phases of data acquisition from the tactile sensors 101 and ultrasound sensors 103.

The analysis process continues sequentially and automatically to the successive points of the multiple points to be analysed 16 by repeating the same working steps F6, F7 and F8. The sequential process of data collection ends at the end of the detection of the last point of the points to be analysed 16.

Once the detection step is over, F9, all the acquired data are processed in order to provide a physical characterization of the biological tissue sample C, as a result, F10, made available through the interface means 15. More specifically, according to an advantageous embodiment of the invention, the analysis by means of the ultrasound sensors 103 is performed by comparing the signal recorded in each single point to be analysed 16 with a suitable reference signal by processing an amplitude factor and a form factor and extracting, on the basis of the differences found in the amplitude and form factors processed, the position and the size of possible inclusions present in the biological tissue sample C. The analysis through the tactile sensors 101 is performed by processing the variation of the force detected by the load cell 1011 along the vertical axis as the indentation depth varies in each single point to be analysed 16 in order to obtain data related to the stiffness of the biological tissue sample C in each single point analysed and to extrapolate from all these values the position and the size of possible inclusions present in the biological tissue sample C. This means that the data acquired by the tactile sensor 101 and by the ultrasound sensor 103 are processed by the control unit 13 independently from each other to determine the same parameters of physical characterization of the biological tissue sample, that is the position of any inclusion and the dimension thereof. The high correlation level between the results obtained confirms the reliability of the results.

According to an advantageous embodiment, the load cell 1011 is a six axis load cell and at each point to be analysed 16 the forces and moments of the forces acting on the load cell 1011 on each of the six axes are processed.

According to a further advantageous embodiment of the apparatus of the invention, the images of the biological tissue sample C acquired by the vision sensors 102 are automatically processed by the control unit 13 according to specific recognition algorithms in order to identify the position and size of any inclusion within the sample C. In this case, also the data acquired by the vision sensors 102 are independently processed by the control unit to determine the same physical characterization parameters of the biological tissue sample determined by the data analysis of the tactile sensor 101 and of the ultrasound sensor 103.

By way of example, in Fig. 6 it is schematically illustrated a view of the biological tissue sample C whose border BE has been recognized and a matrix of points to be analysed 16 has been defined, and finally the results of the analysis are displayed as the position and dimensions of inclusions, I.

In the above described apparatus 1, the data acquired by the tactile sensors 101 and by the vision sensors 102 are processed not only to obtain the physical characterization of the biological tissue sample C, but also to synchronize and regulate the automatic actuation of the moving members 12. In fact, the vision sensors 102 allow to recognize the border of the sample and to define the indentation path, while the tactile sensors 101 allow to determine the end depth of the indentation movement.

The presence of the rod-shaped sensor element 104, rigidly mounted on the load cell 1011, allows to perform both the ultrasound and the tactile analysis simultaneously in the same point, that is to say where is the contact surface 1041.

The pathologist can interact remotely with the apparatus, located in the surgery room, by displaying the images acquired by the vision sensors 102 and the data acquired by the tactile sensors 101 and ultrasound sensors 103, being able to remotely adjust in real time the density of the matrix of points to be analysed 16, the limit force value measured by the tactile sensors 101 for setting the end depth of the indentation and other working parameters of the data acquisition bodies 10. In particular, according to an advantageous embodiment of the invention, output means for showing the results of the analysis performed by the apparatus 1 comprises a haptic interface, for example a glove with transducers or a Phantom-type robotic manipulator, suitable for creating a system of co-decision between the detection and identification algorithms present in the control unit 13 of the apparatus and the remote evaluation through the senses of the pathophysiology doctor who can view the images of the biological sample C and at the same time perceive the tactile sensations through a sort of telepalpation performed by means of the aforementioned haptic interface implemented as a function of the information relating to the physical characterization parameters of the biological sample C (position and dimension of the inclusions I) transmitted by the control unit 13 itself.

All the previously exposed operating steps take place in a time frame of less than one hour, preferably between 20 and 30 minutes, and are therefore performed intraoperatively so that, following the results of the analysis, the surgeon can proceed to operate on the patient's body in view of the results of the analysis.

Moreover, phase F10 of providing the results of the analysis can also advantageously output into an electronic/paper report and integrated with the management systems (for example through the HL7 standard) of the hospital or other structure where the apparatus of the invention is used.

In an alternative embodiment of the apparatus of the invention, not shown, the sensor element 104 can have a free end of a different shape which provides a contact surface 1041 with a greater area, for example comparable to the area of the upper surface of the sample C, arranged to face the flat surface 111 of the platform of the supporting means 11. At the contact surface 1041 of greater size, arrays of ultrasound sensors can be housed, for example of the type comprising a plurality of piezoelectric crystal elements or other piezoelectric material, arranged in a matrix, a comb-like, cubic and/or multilayer arrangement, and tactile sensors arrays. In this embodiment of the apparatus of the invention the sample can be scanned upon a single contact movement of the contact surface on the sample, or with a few contact movements, with the control unit configured to process the plurality of data relating to each ultrasound sensor element and tactile sensor element of the aforementioned sensor arrays.

What above expressed with regard to the structural characteristics of the various components of the apparatus of the invention and in relation to the relative operating modes is purely exemplary and not exhaustive and the above mentioned advantages of the apparatus of the invention certainly remain safe in the presence of variants or modifications of practical application nature, while remaining within the scope of protection defined by the following claims.

## Claims

1. Apparatus (1) for the ex-vivo intraoperative analysis of biological tissue samples (C) resected from a patient during surgery, said apparatus (1) comprising:
- supporting means (11), to which said sample (C) can be stably associated, comprising a platform provided with a flat surface (111) adapted to receive said sample (C),
- data acquisition bodies (10) comprising at least a sensor element (104),
- moving members (12) of said supporting means (11) with respect to said data acquisition bodies (10),
- a control unit (13) for receiving data from said data acquisition bodies (10), for processing them and for controlling said data acquisition bodies (10) and said moving members (12),
**characterized in that** said data acquisition bodies (10) comprise:
- ultrasound sensors (103),
- tactile sensors (101),
- vision sensors (102),
wherein said sensor element (104) provides a free end forming a contact surface (1041) facing said flat surface (111) of the supporting means (11), said contact surface (1041) being suitable for abutting said biological tissue sample (C), said contact surface (1041) being a detection surface of said tactile sensors (101) and the same emission and reception surface of said ultrasound sensors (103), and
wherein said control unit (13) is configured to automatically control said moving members (12) and said ultrasound sensors (103) as a function of data received from said vision sensors (102) and from said tactile sensors (101) in such a way to control the acquisition of data by said ultrasound sensors (103) and tactile sensors (101) and to elaborate the acquired data for a characterization of said biological tissue sample (C).

2. Apparatus (1) for the ex-vivo intraoperative analysis of a biological tissue sample (C) according to claim 1 **characterized in that** said ultrasound sensors (103) are a single ultrasound sensor and said tactile sensors (101) are a single tactile sensor, both associated to the same said sensor element (104) which is a rod-shaped element with a needle-like free end (1042) so that said contact surface (1041) is small in size with respect to the size of said biological tissue sample (C), said control unit (13) being configured to automatically control said moving members (12), ultrasound sensor (103) and tactile sensor (101) in such a way that said contact surface (1041) indents said biological tissue sample (C) in correspondence with a plurality of points to be analysed (16) and to command the acquisition of data by said ultrasound sensor (103) and tactile sensor (101) at each point of said plurality of points to be analysed (16).

3. Apparatus (1) for the ex-vivo intraoperative analysis of a biological tissue sample (C) according to claim 1 **characterized in that** said ultrasound sensors (103) comprise an array of ultrasound transducers and said tactile sensors (101) comprise an array of tactile sensors, both associated to said sensor element (104).

4. Apparatus (1) for the ex-vivo intraoperative analysis of a biological tissue sample (C) according to claim 2 **characterized in that** said vision sensors (102) comprise at least one camera, said control unit (13) being configured to automatic process images detected by said vision sensors (102) in order to identify a border (BE) of said sample (C) to be analysed and said plurality of points to be analysed (16) within an area inside said border (BE).

5. Apparatus (1) for the ex-vivo intraoperative analysis of a biological tissue sample (C) according to the previous claim **characterized in that** said vision sensors (102) comprise a plurality of high-definition cameras, said control unit (13) being configured to process the images detected by said cameras to identify the three-dimensional border (BE) of said sample (C).

6. Apparatus (1) for the ex-vivo intraoperative analysis of a biological tissue sample (C) according to claim 2 **characterized in that** said control unit (13) is configured to, following the definition of said plurality of points to be analysed (16):
- command the automatic indentation in sequence by said sensor element (104) of each of said points to be analysed (16) by actuation of said moving members (12),
- at each of said points to be analysed (16) acquire data by said tactile sensors (101) and ultrasound sensors (103),
- process the data acquired by said tactile sensor (101) and ultrasound sensor (103) independently from each other to determine parameters of physical characterization of said sample (C).

7. Apparatus (1) for the ex-vivo intraoperative analysis of a biological tissue sample (C) according to the previous claim **characterized in that** said control unit (13) is configured to process the data acquired by said vision sensors (102) independently from the data acquired by said tactile sensor (101) and ultrasound sensor (103) to determine the same physical characterization parameters of said sample (C).

8. Apparatus (1) for the ex-vivo intraoperative analysis of a biological tissue sample (C) according to claim 6 or 7, **characterized in that** said physical characterization parameters of said sample (C), determined by said control unit (13) following the processing of said data acquired from said data acquisition bodies (10) consist of the position and dimension of inclusions (I) in said biological tissue sample (C).

9. Apparatus (1) for the ex-vivo intraoperative analysis of a biological tissue sample (C) according to claim 1 **characterized in that** it comprises a portal-like bearing structure (14) that defines therewithin a volume of analysis (VA), said vision sensors (102) being firmly associated with said portal-like bearing structure to shoot said volume of analysis (VA), said sensor element (104) being firmly associated with upper cross-members (141) of said portal-like bearing structure (14) to protrude downwards into said volume of analysis (VA), said supporting means (11) comprising a platform provided with a flat upper surface (111) adapted to receive said sample (C) on it, said platform (11) being housed in said volume of analysis (VA) supported by said moving members (12) consisting of a motorized group with four controlled axes, three of which are of translation and one of rotation.

10. Apparatus (1) for the ex-vivo intraoperative analysis of a biological tissue sample (C) according to the previous claim **characterized in that** said portal-like bearing structure (14) comprises upper cross members (141), lower cross members (142) and uprights (143), said moving members (12) being mounted to said lower cross members (142).

11. Apparatus (1) for the ex-vivo intraoperative analysis of a biological tissue sample (C) according to the previous claim **characterized in that** said moving members (12) comprise longitudinal horizontal slide guides (124), cross horizontal slide guides (123), a lifting mechanism and a rotary cylinder with vertical axis (122), so that the moving members (12) allow said sample (C) to be moved and oriented to any position within the volume of analysis (VA) in which said upper planar surface (111) of the supporting means (11) is kept in a horizontal planar configuration.

12. Apparatus (1) for the ex-vivo intraoperative analysis of a biological tissue sample (C) according to the previous claim **characterized in that** said ultrasound sensors (103) and tactile sensors (101) are included in a same structure fixed below said upper cross-members (141) and comprising a load cell (1011) to which it is rigidly fixed, to protrude downwards in said volume of analysis (VA), a sensor element (104) the free end of which constitutes said contact surface (1041) facing said upper planar surface (111) of the supporting members (11).

13. Apparatus (1) for the ex-vivo intraoperative analysis of a biological tissue sample (C) according to one of the previous claims **characterized in that** output means of the results of the analysis performed by said apparatus (1) comprise a haptic interface, actuated as a function of the information relating to said physical characterization parameters of said biological sample (C) transmitted from said control unit (13).

## Patentansprüche

1. Vorrichtung (1) für die intraoperative *ex*-*vivo*-Analyse von biologischen Gewebeproben (C), die von einem Patienten während einer Operation entnommen wurden, wobei die Vorrichtung (1) umfasst:
- Trägermittel (11), mit denen die Probe (C) stabil verbunden werden kann, mit einer Plattform, die mit einer flachen Oberfläche (111) versehen ist, die geeignet ist, die Probe (C) aufzunehmen,
- Datenerfassungsmittel (10), die mindestens ein Sensorelement (104) umfassen,
- bewegliche Elemente (12) der Trägermittel (11) in Bezug auf die Datenerfassungsmittel (10),
- eine Steuereinheit (13) zum Empfang von Daten von den Datenerfassungsmitteln (10), zu deren Verarbeitung und zur Steuerung der Datenerfassungsmittel (10) und der beweglichen Elemente (12),
**dadurch gekennzeichnet, dass** die Datenerfassungsmittel (10) folgendes umfassen:
- Ultraschallsensoren (103),
- Tastsensoren (101),
- optische Sensoren (102),
wobei das Sensorelement (104) ein freies Ende aufweist, das eine Kontaktfläche (1041) bildet, die der flachen Oberfläche (111) des Trägermittels (11) zugewandt ist, wobei die Kontaktfläche (1041) geeignet ist, an der biologischen Gewebeprobe (C) anzuliegen, wobei die Kontaktfläche (1041) eine Erfassungsfläche der Tastsensoren (101) und die gleiche Sende- und Empfangsfläche der Ultraschallsensoren (103) ist, und
wobei die Steuereinheit (13) so konfiguriert ist, dass sie die sich beweglichen Elemente (12) und die Ultraschallsensoren (103) in Abhängigkeit von den von den Sehsensoren (102) und von den Tastsensoren (101) empfangenen Daten automatisch so steuert, dass die Erfassung von Daten durch die Ultraschallsensoren (103) und die Tastsensoren (101) gesteuert wird und die erfassten Daten für eine Charakterisierung der biologischen Gewebeprobe (C) ausgewertet werden.

2. Vorrichtung (1) für die *ex-vivo* intraoperative Analyse einer biologischen Gewebeprobe (C) nach Anspruch 1, **dadurch gekennzeichnet, dass** die Ultraschallsensoren (103) aus einem einzigen Ultraschallsensor und die Tastsensoren (101) aus einem einzigen Tastsensor bestehen, die beide demselben Sensorelement (104) zugeordnet sind, das ein stabförmiges Element mit einem nadelförmigen freien Ende (1042) ist, so dass die Kontaktfläche (1041) im Verhältnis zur Größe der biologischen Gewebeprobe (C) klein ist, wobei die Steuereinheit (13) so konfiguriert ist, dass sie die beweglichen Elemente (12), den Ultraschallsensor (103) und den Tastsensor (101) automatisch so steuert, dass die Kontaktfläche (1041) die biologische Gewebeprobe (C) in Übereinstimmung mit einer Vielzahl von zu analysierenden Punkten (16) eindrückt und die Erfassung von Daten durch den Ultraschallsensor (103) und den Tastsensor (101) an jedem Punkt der Vielzahl von zu analysierenden Punkten (16) befiehlt.

3. Vorrichtung (1) für die *ex-vivo* intraoperative Analyse einer biologischen Gewebeprobe (C) nach Anspruch 1, **dadurch gekennzeichnet, dass** die Ultraschallsensoren (103) eine Anordnung von Ultraschallwandlern und die Tastsensoren (101) eine Anordnung von Tastsensoren umfassen, die beide dem Sensorelement (104) zugeordnet sind.

4. Vorrichtung (1) für die *ex-vivo* intraoperative Analyse einer biologischen Gewebeprobe (C) nach Anspruch 2, **dadurch gekennzeichnet, dass** die optische Sensoren (102) mindestens eine Kamera umfassen, wobei die Steuereinheit (13) so konfiguriert ist, dass sie die von den optische Sensoren (102) erfassten Bilder automatisch verarbeitet, um eine Grenze (BE) der zu analysierenden Probe (C) und die Vielzahl der zu analysierenden Punkte (16) innerhalb eines Bereichs innerhalb der Grenze (BE) zu identifizieren.

5. Vorrichtung (1) für die *ex-vivo* intraoperative Analyse einer biologischen Gewebeprobe (C) nach dem vorhergehenden Anspruch, **dadurch gekennzeichnet, dass** die optische Sensoren (102) eine Vielzahl von hochauflösenden Kameras umfassen, wobei die Steuereinheit (13) so konfiguriert ist, dass sie die von den Kameras erfassten Bilder verarbeitet, um den dreidimensionalen Rand (BE) der Probe (C) zu identifizieren.

6. Vorrichtung (1) für die *ex-vivo* intraoperative Analyse einer biologischen Gewebeprobe (C) nach Anspruch 2, **dadurch gekennzeichnet, dass** die Steuereinheit (13) so konfiguriert ist, dass sie nach der Definition der mehreren zu analysierenden Punkte (16):
- die automatische Vertiefung eines jeden der zu analysierenden Punkte (16) durch das Sensorelement (104) nacheinander durch Betätigung der beweglichen Elemente (12) zu steuern,
- Erfassen von Daten an jedem der zu analysierenden Punkte (16) durch die Tastsensoren (101) und die Ultraschallsensoren (103),
- die von dem Tastsensor (101) und dem Ultraschallsensor (103) erfassten Daten unabhängig voneinander zu verarbeiten, um physikalischen Charakterisierungsparameter der Probe (C) zu bestimmen.

7. Vorrichtung (1) für die *ex-vivo* intraoperative Analyse einer biologischen Gewebeprobe (C) nach dem vorhergehenden Anspruch, **dadurch gekennzeichnet, dass** die Steuereinheit (13) so konfiguriert ist, dass sie die von den optische Sensoren (102) erfassten Daten unabhängig von den von dem Tastsensor (101) und dem Ultraschallsensor (103) erfassten Daten verarbeitet, um dieselben physikalischen Charakterisierungsparameter der Probe (C) zu bestimmen.

8. Vorrichtung (1) für die *ex-vivo* intraoperative Analyse einer biologischen Gewebeprobe (C) nach Anspruch 6 odr 7, **dadurch gekennzeichnet, dass** die physikalischen Charakterisierungsparameter der Probe (C), die von der Steuereinheit (13) nach der Verarbeitung der von den Datenerfassungseinrichtungen (10) erfassten Daten bestimmt werden, aus der Position und der Größe von Einschlüssen (I) in der biologischen Gewebeprobe (C) bestehen.

9. Vorrichtung (1) für die *ex-vivo* intraoperative Analyse einer biologischen Gewebeprobe (C) nach Anspruch 1, **dadurch gekennzeichnet, dass** sie eine portalartige Tragstruktur (14) umfasst, die darin ein Analysevolumen (VA) definiert, wobei die optischen Sensoren (102) fest mit der portalartigen Tragstruktur verbunden sind, um das Analysevolumen (VA) aufzunehmen, wobei das Sensorelement (104) fest mit oberen Querträgern (141) der portalartigen Tragstruktur (14) verbunden ist, um nach unten in das Analysevolumen (VA) hineinzuragen, wobei die Trägermittel (11) eine Plattform umfassen, die mit einer flachen oberen Oberfläche (111) versehen ist, die geeignet ist die Probe (C) darauf aufzunehmen, wobei die Plattform (11) in dem Analysevolumen (VA) untergebracht ist und von den beweglichen Elementen (12) gestützt wird, die aus einer motorisierten Gruppe mit vier gesteuerten Achsen bestehen, von denen drei translatorisch und eine rotatorisch sind.

10. Vorrichtung (1) für die *ex-vivo* intraoperative Analyse einer biologischen Gewebeprobe (C) nach dem vorhergehenden Anspruch, **dadurch gekennzeichnet, dass** die portalartige Tragstruktur (14) obere Querelemente (141), untere Querelemente (142) und Ständer (143) umfasst, wobei die beweglichen Elemente (12) an den unteren Querelementen (142) angebracht sind.

11. Vorrichtung (1) für die *ex-vivo* intraoperative Analyse einer biologischen Gewebeprobe (C) nach dem vorhergehenden Anspruch, **dadurch gekennzeichnet, dass** die beweglichen Elemente (12) längs verlaufende horizontale Gleitführungen (124), quer verlaufende horizontale Gleitführungen (123), einen Hebemechanismus und einen Drehzylinder mit vertikaler Achse (122) umfassen, so dass die beweglichen Elemente (12) es ermöglichen, die Probe (C) in jede Position innerhalb des Analysevolumens (VA) zu bewegen und auszurichten, in der die obere ebene Fläche (111) des Trägermittels (11) in einer horizontalen ebenen Konfiguration gehalten wird.

12. Vorrichtung (1) für die *ex-vivo* intraoperative Analyse einer biologischen Gewebeprobe (C) nach dem vorhergehenden Anspruch, **dadurch gekennzeichnet, dass** die Ultraschallsensoren (103) und die Tastsensoren (101) in einer gleichen Struktur enthalten sind, die unterhalb der oberen Querträger (141) befestigt ist und eine Kraftmesszelle (1011) umfasst, an der sie starr befestigt ist, ein Sensorelement (104), dessen freies Ende die Kontaktfläche (1041) bildet, die der oberen ebenen Fläche (111) der Tragelemente (11) zugewandt ist, um nach unten in das Analysevolumen (VA) zu ragen.

13. Vorrichtung (1) für die *ex-vivo* intraoperative Analyse einer biologischen Gewebeprobe (C) nach einem der vorhergehenden Ansprüche, **dadurch gekennzeichnet, dass** die Ausgabemittel für die Ergebnisse der von der Vorrichtung (1) durchgeführten Analyse eine haptische Schnittstelle umfassen, die in Abhängigkeit von den von der Steuereinheit (13) übertragenen Informationen bezüglich der physikalischen Charakterisierungsparameter der biologischen Probe (C) betätigt wird.

## Revendications

1. Appareil (1) pour l'analyse intra-opératoire *ex-vivo* d'échantillons de tissus biologiques (C) réséqués chez un patient pendant une intervention chirurgicale, ledit appareil (1) comprenant :
- des moyens de support (11), auxquels ledit échantillon (C) peut être associé de manière stable, comprenant une plate-forme munie d'une surface plane (111) adaptée pour recevoir ledit échantillon (C),
- des organes d'acquisition de données (10) comprenant au moins un élément capteur (104),
- des organes de mouvement (12) desdits moyens de support (11) par rapport auxdits organes d'acquisition de données (10),
- une unité de commande (13) pour recevoir des données provenant desdits organes d'acquisition de données (10), pour les traiter et pour commander lesdits organes d'acquisition de données (10) et lesdits organes de mouvement (12),
**caractérisé en ce que** lesdits organes d'acquisition de données (10) comprennent:
- des capteurs à ultrasons (103),
- des capteurs tactiles (101),
- des capteurs de vision (102),
dans lesquels ledit élément de capteur (104) fournit une extrémité libre formant une surface de contact (1041) faisant face à ladite surface plate (111) du moyen de support (11), ladite surface de contact (1041) étant appropriée pour venir en butée contre ledit échantillon de tissu biologique (C), ladite surface de contact (1041) étant une surface de détection desdits capteurs tactiles (101) et la même surface d'émission et de réception desdits capteurs à ultrasons (103), et
dans lequel ladite unité de commande (13) est configurée pour commander automatiquement lesdits organes de mouvement (12) et lesdits capteurs à ultrasons (103) en fonction des données reçues desdits capteurs de vision (102) et desdits capteurs tactiles (101) de manière à commander l'acquisition de données par lesdits capteurs à ultrasons (103) et capteurs tactiles (101) et à élaborer les données acquises pour une caractérisation dudit échantillon de tissu biologique (C).

2. Appareil (1) pour l'analyse intra-opératoire *ex-vivo* d'un échantillon de tissu biologique (C) selon la revendication 1, **caractérisé en ce que** lesdits capteurs à ultrasons (103) consistent en un seul capteur à ultrasons et lesdits capteurs tactiles (101) consistent en un seul capteur tactile, tous deux associés au même élément de capteur (104) qui est un élément en forme de tige avec une extrémité libre en forme d'aiguille (1042) de sorte que ladite surface de contact (1041) est de petite taille par rapport à la taille dudit échantillon de tissu biologique (C), ladite unité de commande (13) étant configurée pour commander automatiquement lesdits organes de mouvement(12), le capteur à ultrasons (103) et le capteur tactile (101) de telle sorte que ladite surface de contact (1041) entaille ledit échantillon de tissu biologique (C) en correspondance de une pluralité de points à analyser (16) et pour commander l'acquisition de données par ledit capteur à ultrasons (103) et ledit capteur tactile (101) en chaque point de ladite pluralité de points à analyser (16).

3. Appareil (1) pour l'analyse intra-opératoire *ex-vivo* d'un échantillon de tissu biologique (C) selon la revendication 1, **caractérisé en ce que** lesdits capteurs à ultrasons (103) comprennent un réseau de transducteurs à ultrasons et lesdits capteurs tactiles (101) comprennent un réseau de capteurs tactiles, tous deux associés audit élément capteur (104).

4. Appareil (1) pour l'analyse intra-opératoire *ex-vivo* d'un échantillon de tissu biologique (C) selon la revendication 2, **caractérisé en ce que** lesdits capteurs de vision (102) comprennent au moins une caméra, ladite unité de commande (13) étant configurée pour traiter automatiquement les images détectées par lesdits capteurs de vision (102) afin d'identifier un bord (BE) dudit échantillon (C) à analyser et ladite pluralité de points à analyser (16) dans une zone à l'intérieur de ledit bord (BE).

5. Appareil (1) pour l'analyse intra-opératoire *ex-vivo* d'un échantillon de tissu biologique (C) selon la revendication précédente, **caractérisé en ce que** lesdits capteurs de vision (102) comprennent une pluralité de caméras haute définition, ladite unité de commande (13) étant configurée pour traiter les images détectées par lesdites caméras afin d'identifier le bord tridimensionnel (BE) dudit échantillon (C).

6. Appareil (1) pour l'analyse intra-opératoire *ex-vivo* d'un échantillon de tissu biologique (C) selon la revendication 2, **caractérisé en ce que** ladite unité de commande (13) est configurée pour, suite à la définition de ladite pluralité de points à analyser (16):
- commander l'indentation automatique en séquence par ledit élément capteur (104) de chacun desdits points à analyser (16) par actionnement desdits organes de mouvement (12),
- à chacun desdits points à analyser (16), acquérir des données par lesdits capteurs tactiles (101) et capteurs à ultrasons (103),
- traiter les données acquises par ledit capteur tactile (101) et ledit capteur à ultrasons (103) indépendamment l'un de l'autre pour déterminer des paramètres de caractérisation physique dudit échantillon (C).

7. Appareil (1) pour l'analyse intra-opératoire *ex-vivo* d'un échantillon de tissu biologique (C) selon la revendication précédente, **caractérisé en ce que** ladite unité de commande (13) est configurée pour traiter les données acquises par lesdits capteurs de vision (102) indépendamment des données acquises par ledit capteur tactile (101) et ledit capteur à ultrasons (103) pour déterminer les mêmes paramètres de caractérisation physique dudit échantillon (C).

8. Appareil (1) pour l'analyse intra-opératoire *ex-vivo* d'un échantillon de tissu biologique (C) selon la revendication 6 ou 7, **caractérisé en ce que** lesdits paramètres de caractérisation physique dudit échantillon (C), déterminés par ladite unité de commande (13) après le traitement desdites données acquises à partir desdits corps d'acquisition de données (10) consistent en la position et la dimension des inclusions (I) dans ledit échantillon de tissu biologique (C).

9. Appareil (1) pour l'analyse intra-opératoire *ex-vivo* d'un échantillon de tissu biologique (C) selon la revendication 1, **caractérisé en ce que** il comprend une structure porteuse en forme de portail (14) qui définit à l'intérieur un volume d'analyse (VA), lesdits capteurs de vision (102) étant fermement associés à ladite structure porteuse en forme de portail pour filmer ledit volume d'analyse (VA), ledit élément de capteur (104) étant fermement associé à des traverses supérieures (141) de ladite structure de support en forme de portail (14) pour faire saillie vers le bas dans ledit volume d'analyse (VA), lesdites moyens de support comprenant une plate-forme munie d'une surface supérieure plate (111) adaptée pour recevoir ledit échantillon (C) sur celle-ci, ladite plate-forme (11) étant logée dans ledit volume d'analyse (VA) supportée par lesdits organes de mouvement (12) consistant en un groupe motorisé avec quatre axes commandés, dont trois sont de translation et un de rotation.

10. Appareil (1) pour l'analyse intra-opératoire *ex-vivo* d'un échantillon de tissu biologique (C) selon la revendication précédente, **caractérisé en ce que** ladite structure porteuse en forme de portail (14) comprend des traverses supérieures (141), des traverses inférieures (142) et des montants (143), lesdits organes de mouvement (12) étant montés sur lesdites traverses inférieures (142).

11. Appareil (11) pour l'analyse intra-opératoire *ex-vivo* d'un échantillon de tissu biologique (C) selon la revendication précédente, **caractérisé en ce que** lesdits organes de mouvement (12) comprennent des guides de glissement horizontaux longitudinaux (124), des guides de glissement horizontaux transversaux (123), un mécanisme de levage et un cylindre rotatif à axe vertical (122), de sorte que les organes de mouvement (12) permettent audit échantillon (C) d'être déplacé et orienté vers n'importe quelle position à l'intérieur du volume d'analyse (VA) dans lequel ladite surface plane supérieure (111) du moyen de support (11) est maintenue dans une configuration plane horizontale.

12. Appareil (1) pour l'analyse intra-opératoire *ex-vivo* d'un échantillon de tissu biologique (C) selon la revendication précédente, **caractérisé en ce que** lesdits capteurs à ultrasons (103) et capteurs tactiles (101) sont inclus dans une même structure fixée sous lesdites traverses supérieures (141) et comprenant une cellule de charge (1011) à laquelle elle est rigidement fixée, pour faire saillie vers le bas dans ledit volume d'analyse (VA), un élément capteur (104) dont l'extrémité libre constitue ladite surface de contact (1041) faisant face à ladite surface plane supérieure (111) des éléments de support (11).

13. Appareil (1) pour l'analyse intra-opératoire *ex-vivo* d'un échantillon de tissu biologique (C) selon n'importe laquelle des revendications antérieurs, **caractérisé en ce que** les moyens de sortie des résultats de l'analyse effectuée par ledit appareil (1) comprennent une interface haptique, actionnée en fonction des informations relatives auxdits paramètres de caractérisation physique dudit échantillon biologique (C) transmises par ladite unité de commande (13).
